# EUROPÄISCHE PATENTSCHRIFT

(11) **EP 2 354 115 B1**
(45) Veröffentlichungstag und Bekanntmachung des Hinweises auf die Patenterteilung: **24.08.2016**
(21) Anmeldenummer: 11152683.6
(22) Anmeldetag: 31.01.2011
(51) Int. Cl.: C07C 68/06, C07C 68/08, C07C 69/96

(54) **Verfahren zur Herstellung von Dialkylcarbonaten**
Method for producing dialkyl carbonates
Procédé destiné à la fabrication de carbonates de dialkyle

(30) Priorität: 03.02.2010 DE 102010006657
(43) Veröffentlichungstag der Anmeldung: 10.08.2011
(73) Patentinhaber: Covestro Deutschland AG, 51373 Leverkusen (DE)
(72) Erfinder: Ooms, Pieter, Krefeld, 47800 (DE); Risse, Friedhelm, 50739, Köln (DE); Düx, Andre, 50321, Brühl (DE); Buchaly, Carsten, 40233, Düsseldorf (DE); Pancur, Thomas, 24161, Altenholz (DE); Susanto, Arthur, 50670, Köln (DE); Ronge Georg, 40549, Düsseldorf (DE); Vanden Eynde, Johan, 9052, Zwijnaarde (BE); Wuytack, Wim, 9240, Zele (BE)
(74) Vertreter: Levpat

(56) Entgegenhaltungen:
- EP-A1- 1 927 583
- US-A1- 2003 023 109
- US-A1- 2007 197 816

## Beschreibung

Die vorliegende Erfindung betrifft ein kontinuierliches Verfahren zur Herstellung von niederen Dialkylcarbonaten als Hauptprodukt und Alkylenglykol als Nebenprodukt durch Umesterung eines zyklischen Alkylencarbonats (z.B. Ethylen- oder Propylencarbonat) mit niederen Alkylalkoholen in Gegenwart eines Katalysators sowie die erforderliche Aufreinigung des Dialkylcarbonats in einem nachfolgenden Verfahrensschritt. Zur Optimierung der Wirtschaftlichkeit und Energieeffizienz des Verfahrens werden zusätzliche Vorrichtungen zur Zwischenerhitzung der apparateinternen Flüssigkeitströme eingesetzt.

Die Herstellung von Dialkylcarbonaten aus zyklischem Alkylencarbonat und Alkylalkohol, bei der gleichzeitig Alkylenglykol als Nebenprodukt entsteht, ist bekannt und vielfach beschrieben worden. In U S-6,930,195 B wurde diese katalysierte Umesterungsreaktion als zweistufige Gleichgewichtsreaktion beschrieben. In der ersten Reaktionsstufe reagiert das zyklische Alkylencarbonat mit Alkylalkohol zu Hydroxy-alkylcarbonat als Zwischenprodukt. Das Zwischenprodukt wird dann in der zweiten Reaktionsstufe mit Hilfe von Alkylalkohol umgesetzt zu den Produkten: Dialkylcarbonat und Alkylenglykol.

Für die technische Realisierung des Dialkylcarbonat-Herstellungsverfahrens hat sich die Verwendung einer reaktiven Destillationskolonne (im Folgenden auch Umesterungskolonne genannt), die unter anderem bereits in EP 530 615 A, EP 569 812 A und EP 1 086 940 A beschrieben wurde, als besonders günstig erwiesen. In EP 569 812 A wird das zyklische Alkylencarbonat in den oberen Teil der Umesterungskolonne und der Dialkylcarbonat enthaltende Alkylalkohol in den mittleren oder unteren Teil der Umesterungskolonne kontinuierlich eingeführt. Zusätzlich wird unterhalb der Einführung des Dialkylcarbonat enthaltenden Alkylalkohols nahezu reiner Alkylalkohol eingeführt. Eine Substanz wird als nahezu rein im Sinne dieser Erfindung bezeichnet, wenn sie weniger als 2 Gew.-%, bevorzugt weniger als 1 Gew.-%, Verunreinigungen enthält. Das Schwersiedergemisch, welches das hergestellte Alkylenglykol als Nebenprodukt beinhaltet, wird am Sumpf der Umesterungskolonne kontinuierlich abgezogen. Das Leichtsiedergemisch, welches das hergestellte Dialkylcarbonat beinhaltet, wird am Kopf der Umesterungskolonne als Dialkylcarbonat-Alkylalkohol-Gemisch abgezogen und einem weiteren Aufreinigungsschritt unterworfen.

Die Destillationskolonne für die Aufreinigung des Dialkylcarbonat-Alkylalkohol-Gemisches wird bei einem höheren Druck als dem in der Umesterungskolonne herrschenden betrieben, so dass ein weiteres Dialkylcarbonat-Alkylalkohol-Gemisch mit einem niedrigeren Dialkylcarbonat-Anteil am Kolonnenkopf dieser Destillationskolonne abgezogen werden kann. Das Dialkylcarbonat als Hauptprodukt wird am Sumpf dieser Aufreinigungskolonne mit hoher Reinheit gewonnen.

Für die Entwicklung eines wirtschaftlich attraktiven Herstellungsverfahrens für Dialkylcarbonate spielen viele Faktoren eine wichtige Rolle. Die meisten Literaturquellen beschäftigen sich mit den Reaktionsparametern, wie z.B. Umsatz, Selektivität oder auch Produktreinheit. Seltener wurde die Energieeffizienz des Verfahrens thematisiert (z.B. in EP 569 812 A, JP2003-104937, WO 2007/096340, WO 2007/096343), obwohl dieser Faktor nicht unerheblich zu der wirtschaftlichen Attraktivität des Verfahrens beiträgt. Daher werden in dieser Erfindung Maßnahmen eingeführt, um die Energieeffizienz des Verfahrens zu erhöhen.

In EP 569 812 A wird der Energieeinsatz in der Herstellung des Dialkylcarbonates dadurch reduziert, dass viele verfahrensinterne Ströme nicht kondensiert sondern als dampfförmige Ströme geführt werden.

In WO 2007/096340 wird ein Verfahren beschrieben, in dem Alkylencarbonat aus Alkylenoxid und CO₂ erzeugt und anschließend das Alkylencarbonat mit Alkylalkohol zu Dialkylcarbonat und Alkylenglykol umgesetzt wird, wobei das im zweiten Schritt entstehende Gemisch, welches Dialkylcarbonat und Alkylenglykol enthält, aufgereinigt wird. Die Reaktion zum Alkylencarbonat ist exotherm und der entsprechende Alkylencarbonat-Produktstrom wird zur Aufwärmung des Dialkylcarbonat-Alkylenglykol-Produktstromes in der Aufreinigung eingesetzt.

In WO 2007/096343 wird das in einer Umesterungskolonne aus Alkylencarbonat und Alkylalkohol entstehende Gemisch aus Dialkylcarbonat und Alkylalkohol mittels Extraktivdestillation gereinigt, wobei Alkylencarbonat als Extraktionsmittel dient. Nachdem das Dialkylcarbonat destillativ vom Extraktionsmittel getrennt wurde, wird mit dem heißen Sumpfablauf dieser Kolonne, der das Extraktionsmittel enthält, der der Umesterungskolonne zugeführte Alkylalkohol erwärmt.

In JP 2003-104937 werden verschiedene Verfahrensvarianten zur Aufarbeitung eines Ethylencarbonat-Ethylenglykol-Gemisches und Bereitstellung des gereinigten Ethylencarbonates für das Verfahren zur Herstellung von Dimethylcarbonat auch unter dem Gesichtspunkt des Energieverbrauchs betrachtet.

Keine der genannten Schriften beschreibt aber Verfahren oder Vorgehensweisen, wie unter Beibehaltung der Qualität des Hauptproduktes (Dialkylcarbonat) sowie des Nebenproduktes (Alkylenglykol) die Umsetzung von Alkylencarbonat mit Alkylalkohol in der Umesterungskolonne besonders energieeffizient durchgeführt werden kann. Daher werden in dieser Erfindung Maßnahmen eingeführt, um die Energieeffizienz in diesemVerfahrensschritt zu erhöhen.

Es bestand daher der Bedarf nach einem Verfahren, das eine höhere Energieeffizienz in der Umesterungskolonne aufweist bei gleichbleibender Qualität des Dialkylcarbonates und des Alkylenglykols.

Es wurde nun herausgefunden, dass die Energieeffizienz dadurch erhöht werden kann, indem der Bedarf an Wärmeenergie auf dem Temperaturniveau T_{SV,} welches zum Betrieb des Sumpfverdampfers der Umesterungskolonne benötigt wird, besonders einfach und günstig bei gleich bleibender Produktqualität durch die Verwendung einer zusätzlichen technischen Vorrichtung zur Zwischenerhitzung in der Umesterungskolonne verringert werden kann, wobei die freiwerdende Kondensationswärme des Kopfkondensators oder die Wärme aus dem Sumpfablauf der Dialkylcarbonat-Aufreinigungskolonne zurückgewonnen und direkt oder indirekt, ganz oder teilweise für die Zwischenerhitzung verwendet wird.

Bedingt durch die niedrigere Temperatur des internen Stoffstromes der Umesterungskolonne im Vergleich zur Sumpftemperatur dieser Kolonne, kann für die Zwischenerhitzung Wärmeenergie auf dem Temperaturniveau T_{Z} mit T_{Z} < T_{SV} eingesetzt werden. Dieses Konzept führt insgesamt zu einer Verringerung des Verbrauchs an Wärmeenergie auf einem Temperaturniveau größer oder gleich T_{SV,} weil nun die bei einer Kondensation oder bei der Abkühlung eines Stoffstromes anfallende Wärmeenergie auf einem Temperaturniveau kleiner T_{SV} , die auch aus anderen chemischen Herstellverfahren stammen kann, sinnvoll genutzt werden und die Menge an im Allgemeinen kostenintensiverer Wärmeenergie auf einem Temperaturniveau größer oder gleich T_{SV} reduziert werden kann.

Am Kopfkondensator der Dialkylcabonat-Aufreinigungskolonne lässt sich aus der freiwerdenden Kondensationswärme niedrigstufiger Dampf gewinnen. Es wurde nun gefunden, dass dieser niedrigstufige Dampf geeignet ist, um zum Beispiel den Feed zur Dialkylcarbonat-Aufreinigungskolonne vorzuwärmen, den Dialkylcarbonat enthaltenden Alkylalkoholstrom zur Umesterungskolonne zu verdampfen oder zur Zwischenerhitzung des kolonneninternen Flüssigkeitstromes in der Umesterungskolonne zu verwenden oder eine Kombination der genannten Möglichkeiten zu realisieren.

Die am Kopfkondensator oder aus dem Sumpfablauf der Dialkylcabonat-Aufreinigungskolonne gewonnene Wärmeenergie oder auch die in anderen chemischen Herstellverfahren gewonnene Wärmeenergie auf dem Temperaturniveau T_{Z} kann entweder direkt oder indirekt dem Zwischenerhitzer zugeführt werden. Bei direkter Zuführung erwärmt der Stoffstrom, der kondensiert oder abgekühlt werden soll, mittels des Zwischenerhitzers den kolonneninternen Strom der Umesterungskolonne. Bei indirekter Zuführung erwärmt der zu kondensierende oder abzukühlende Stoffstrom über die Vermittlung eines oder mehrerer Wärmeträgermedien den kolonneninternen Strom. Als Wärmeträgermedien kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl™, Marlotherm^{®}). Besonders bevorzugte Wärmeträgermedien sind Wasser oder Wasserdampf.

Es stellt sich weiterhin überraschenderweise heraus, dass, im Falle einer Zwischenerhitzung in der Umesterungskolonne, diese bevorzugt zwischen dem Kolonnensumpf und der untersten Einführungsstelle für Reaktanden platziert werden kann. Dadurch kann der Gewichtsanteil an nicht umgesetztem Alkylencarbonat im Sumpfprodukt der Umesterungskolonne weiterhin unter 1000 ppm, bevorzugt unter 500 ppm gehalten werden. Der niedrigstufige Dampf für den Zwischenerhitzer wird, wie bereits erwähnt, am Kopfkondensator der Dialkylcarbonat-Aufreinigungskolonne erzeugt.

Der Zwischenerhitzer kann in die Umesterungskolonne integriert oder als separater Zwischenerhitzer außerhalb der Kolonne ausgeführt sein. Die Ausführung des innen- oder außenliegenden Zwischenerhitzers kann dabei sowohl 1-stufig als auch mehrstufig (d. h. ein oder mehrere Wärmetauscher) erfolgen. Für den Zwischenerhitzer sind darüber hinaus erfindungsgemäß verschiedene Konstruktionen möglich, wie z.B. integrierte Heizregister oder Heizschlangen bei innenliegender Ausführung sowie beispielsweise Plattenwärmetauscher oder Rohrbündelwärmetauscher bei außenliegender Ausführung. Solche Konstruktionen sind dem Fachmann bekannt.

In der bevorzugten innenliegenden Ausführungsform weist der Zwischenerhitzer der Destillationskolonne zur Aufreinigung des Dialkylcarbonates bevorzugt eine Länge von 100 bis 10000 mm auf und das Verhältnis von Durchmesser des Zwischenerhitzers zum Kolonnendurchmesser liegt bevorzugt bei 0,1 bis 1. Weiterhin bevorzugt weist der Zwischenerhitzer eine Wärmeübertragungsfläche von 1 bis 5000 m² auf.

Die Verdampfung des Dialkylcarbonat enthaltenden Alkylalkoholstroms kann ein- oder mehrstufig mittels Wärmetauscher wie beispielsweise Platten- oder Rohbündelwärmetauscher erfolgen.

Durch die Verringerung des Verbrauchs an Wärmeenergie auf dem hohen Temperaturniveau T_{SV} unter gleichzeitiger Beibehaltung der hohen Produktqualität ergibt sich durch das erfindungsgemäße Verfahren ein signifikanter ökonomischer Vorteil.

Im Rahmen der Erfindung gereinigte Dialkylcarbonate sind bevorzugt solche der allgemeinen Formel (I) 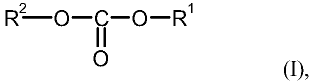 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Die Dialkylcarbonate werden bevorzugt aus zyklischen Alkylencarbonaten mit der Formel (II) hergestellt: 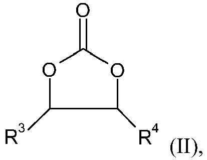 wobei in der Formel R³ und R⁴ unabhängig voneinander Wasserstoff, substituiertes oder nicht substituiertes **C₁-C₄-Alkyl,** substituiertes oder nicht substituiertes **C₂-C₄-Alkenyl** oder substituiertes oder nicht substituiertes **C₆-C₁₂-Aryl** und R³ und R⁴ gemeinsam mit den beiden Dreiring-C-Atomen einen gesättigten carbozyklischen Ring mit 5 - 8 Ringgliedern bedeuten können.

Die zyklischen Alkylencarbonate werden mit Alkoholen der Form

R⁵-OH

umgesetzt, wobei R⁵ ein geradkettiges oder verzweigtes **C₁-C₄-Alkyl** bedeutet.

Zur Erzeugung der Dialkylcarbonate verwendete Umesterungskatalysatoren sind die dem Fachmann bekannt, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US 3,642,858 A, US 3 803 201 A, EP 1 082 A). Für den Fall des Einsatzes der Alkoholate können diese auch in situ durch Einsatz der elementaren Alkalimetalle und dem umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder lodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Solche Alkalimetallverbindungen (gegebenenfalls in situ gebildet aus den freien Alkalimetallen) werden in Mengen von 0,001 bis 2 Gew.-%, bevorzugt 0,003 bis 1,0 Gew.-%, besonders bevorzugt 0,005 bis 1,0 Gew.-%, bezogen auf das umzusetzende Reaktionsgemisch, eingesetzt.

Es ist möglich, solchen Alkalimetallverbindungen gegebenenfalls komplexierende Stoffe zuzusetzen. Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt in 1 bis 100 mol-%, bezogen auf die Alkalimetallverbindung, eingesetzt.

Als Katalysatoren für die Herstellung von Dialkylcarbonate sind ferner Thallium-I- und Thallium-lll-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt TI-(I)-oxid, TI-(I)-hydroxid, TI-(I)-carbonat, TI-(I)-acetat, TI-(111)-acetat, TI-(I)-fluorid, TI-(I)-formiat, TI-(I)-nitrat, TI-(I)-naphtenat und TI-(I)-methylat (EP 1 083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch. Im Herstellungsverfahren können weiterhin stickstoffhaltige Basen als Katalysatoren eingesetzt werden (US 4 062 884). Genannt seien beispielsweise sek. oder tert. Amine wie Triethylamin, Tributylamin, Methyldibenzylamin, Dimethylcyclohexylamin u.a..

Die eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch. Als Katalysatoren sind ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519).

Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenylarsino)ethan, Triphenylantimon, Diphenylsulfid, Diphenyldisulfid, Diphenylselenid, Tetraphenylphosphoniumhalogenid (Cl, Br, J), Tetraphenylarsoniumhalogenid (Cl, Br, J), Triphenylsulfoniumhalogenid (Cl, Br) usw.

Die bevorzugten Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin als Katalysatoren einsetzbar sind Verbindungen des Zinns, Titans oder Zirkoniums (US-4,661,609 A). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethyl-hexanoate, Zirkoniumalkoxide (Methyl, Ethyl, Butyl), Zirkonium(1V)halogenide (F, Cl, Br, J), Zirkoniumnitrate, Zirkoniumacetylacetonat, Titanalkoxide (Methyl, Ethyl, Isopropyl), Titanacetat, Titanacetylacetonat usw.

Die bevorzugt einsetzbaren Mengen dieser Katalysatoren betragen 0,1 bis 10 Gew.-%, bevorzugt 0,1 bis 5 Gew.-%, bezogen auf das Gesamtgemisch.

Im Herstellungsverfahren können weiterhin bifunktionelle Katalysatoren der Formel (III)

[AₐX_{b}]ₘ • [B_{c}Y_{d}]ₙ (III)

eingesetzt werden. In diesen bifunktionellen Katalysatoren wird das molare Verhältnis der beiden in eckigen Klammern stehenden Komponenten durch die Indices m und n ausgedrückt. Diese Indices können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indices a und b stellen unabhängig voneinander ganze Zahlen von 1-5 dar; die Indices C und d bedeuten unabhängig voneinander ganze Zahlen von 1-3, wobei den Forderungen der Valenzen der Kationen und Anionen zur Bildung solcher neutraler Salze zu entsprechen ist. Des weiteren bedeuten in (III) A das Kation eines Metalles, das der dritten Periode und Gruppe IIa, der vierten Periode und Gruppe IIa, IVa-VIIIa, Ib oder IIb, der fünften Periode und Gruppe IIa, IVa-VIIa oder IVb bzw. der sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, lodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, lodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, lodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formel (IV) 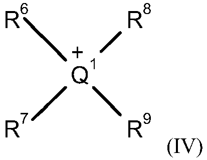 in Frage, worin
Q¹ für N, P, As oder Sb steht und
R⁶, R⁷, R⁸ und R⁹ unabhängig voneinander geradkettiges oder verzweigtes C₁-C₁₈ oder C₇-C₁₂-Aralkyl sind und einer der Reste R⁶-R⁹ auch C₆ - C₁₂ sein kann. In besonders bevorzugter Weise ist B ein Kation der Formel (V) 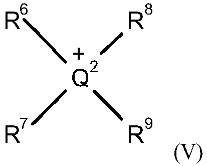 worin,
   Q² für N oder P, bevorzugt für N steht.

In ganz besonders bevorzugter Weise treten im Rahmen der Formeln (IV) bzw. (V) an die Stelle der Reste R⁶, R⁷, R⁸ und R⁹ die Reste R¹⁶, R¹⁷, R¹⁸ bzw. R¹⁹, die unabhängig voneinander geradkettiges oder verzweigtes C₁-C₁₈-Alkyl oder C₇-C₈-Aralkyl bedeuten und einer der Reste R¹⁶ bis R¹⁹ auch Phenyl sein kann. In weiterhin ganz besonders bevorzugter Weise treten an die Stelle der Reste R¹⁶, R¹⁷, R¹⁸ bzw. R¹⁹, die Reste R²⁶, R²⁷, R²⁸ bzw. R²⁹, die unabhängig voneinander C₁-C₈-Alkyl oder Benzyl bedeuten und einer der Reste R²⁶ bis R²⁹ auch Phenyl sein kann.

Geradkettiges oder verzweigtes C₁-C₁₈-Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthylethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (III) ist ein Halogenidion, wie Fluorid, Chlorid, Bromid oder lodid, bevorzugt Bromid oder lodid, besonders bevorzugt lodid. Es kann jedoch auch die Bedeutung von anderen unter X genannten Anionen besitzen, wenn im konkreten Fall das Anion X Bromid oder lodid ist.

Der bifunktionelle Katalysator der Formel (III) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Solche Katalysatoren können homogen gelöst auf den Kopf der Kolonne gegeben werden, wobei als Lösungsmittel Alkylencarbonat, Alkylenglykol, Alkohol oder Dialkylcarbonat, also systemeigene Lösungsmittel, zur Anwendung gelangen. Es ist selbstverständlich möglich, nicht lösliche Umesterungskatalysatoren einzusetzen, die auf den Zwischenböden oder inmitten der Füllkörper angeordnet sind. In einem solchen Fall kann die Dosierung eines gelösten Katalysators über (2) entfallen. Geeignete heterogene Katalysatoren sind beispielsweise:

lonentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein (US 4,062,884 A, US 4,691,041 A, EP 298 167 A). Weiterhin genannt seien Alkali- oder Erdalkalisilikate, imprägniert auf Siliciumdioxidträgern, sowie Ammonium-ausgetauschte Zeolithe.

Das Herstellungsverfahren kann kontinuierlich oder diskontinuierlich durchgeführt werden. Bevorzugt ist eine kontinuierliche Fahrweise.

Im Verfahren werden die zyklische(n) Alkylencarbonatverbindung(en) und der oder die Alkylalkohol(e) bevorzugt im Molverhältnis von 1 : 0.1 bis 1 : 40, besonders bevorzugt von 1 : 1.0 bis 1 : 30, ganz besonders bevorzugt von 1 : 2.0 bis 1 : 20 eingesetzt. Dabei berücksichtigt das angegebene Molverhältnis nicht die Rückführung von zyklischer Alkylencarbonatverbindung oder Alkylalkohol in die Umesterungskolonne über einen oder mehrere Kopfkondensator(en) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkylalkohols angeordnet ist, in die Kolonne eingebracht. Alternativ kann der Katalysator auch separat beispielsweise im Alkylalkohol, im Alkylenglykol oder in einem geeigneten inerten Lösungsmittel gelöst, zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Der Umsatz von Alkylencarbonat und Alkylalkohol zu Dialkylcarbonat und Alkylenglykol findet fast vollständig in einer Umesterungskolonne statt. In bevorzugten Ausführungsformen des Verfahrens zur Herstellung von Dialkylcarbonat kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Ein geeignetes Kolonnendesign der im Verfahren eingesetzten Destillations- und/oder Reaktionskolonnen, welches sowohl die Festlegung der Kolonnenhöhe, des Kolonnendurchmessers, die Auswahl der Kolonneneinbauten als auch die Dimensionierung der Zulauf- und Entnahmeleitungen umfasst, ist dem Fachmann bekannt und kann der einschlägigen Literatur (z.B. Distillation Design, Henry Z. Kister, Mc Graw Hill; Distillation Operation, Henry Z. Kister, Mc Graw Hill; Perry's Chemical Engineering Handbook; Perry & Green) entnommen werden.

Hinsichtlich der Kondensation im Kopfkondensator sind unterschiedliche Ausführungsformen denkbar. Als Kopfkondensatoren eignen sich beispielsweise Rohrbündel- oder Plattenwärmetauscher. Das Verhältnis d1/D1 von Durchmesser der Dampfleitung von der Kolonne zum Kondensator (d1) zu Kolonnendurchmesser der Destillationskolonne (D1) liegt bevorzugt im Bereich von 0,2 bis 1,0, besonders bevorzugt im Bereich von 0,5 bis 1. In einer besonders bevorzugten Ausführungsform kann der Kopfkondensator in die Destillationskolonne integriert sein, so dass zwischen Destillationskolonne und Kopfkondensator keine Dampfleitung erforderlich ist. Das Verhältnis d₁/D₁ beträgt in diesem Fall 1. Dabei kann der Kolonnenquerschnitt nach Eintritt in den Kopfkondensator unter Umständen auch dem Kondensationsfortschritt angepasst werden.

Bei einigen Kondensatorformen kann es von Vorteil sein, den Kolonnenquerschnitt variabel zu gestalten. Ist die Führung der zu kondensierenden Dämpfe beispielsweise von unten nach oben, so nimmt die Dampfmenge nach oben hin ab. Durch eine Reduktion des Kolonnendurchmessers in Richtung Kolonnenkopf, wird der für den Dampf verfügbare Kolonnenquerschnitt der nach oben hin abnehmenden Dampfmenge angepasst. Dabei muss die Entnahme der nicht kondensierten Dämpfe nicht zwangsläufig oben erfolgen. Wird beispielsweise eine Konstruktion gewählt, bei der ein Platten- oder Rohrbündel von oben in die Kolonne einfügt wird, so kann sich die Entnahme der nicht kondensierten Dämpfe auch seitlich befinden.

Die Umesterungskolonne enthält bevorzugt wenigstens einen Verstärkungsteil im oberen Teil der Kolonne und wenigstens eine Reaktionszone unterhalb des Verstärkungsteils. Der Verstärkungsteil weist unabhängig 0 bis 30, bevorzugt 0,1 bis 30 theoretische Stufen auf.

Die Umesterungskolonne weist unterhalb einer Reaktionszone wenigstens ein Abtriebsteil auf, das über 0 bis 20, bevorzugt 1 bis 10 theoretische Stufen verfügt.

Die Umesterungskolonne ist mit einem oder mehreren Sumpfverdampfer(n) ausgestattet. Zusätzlich wird ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt.

Das oder die Verstärkungsteil(e) sind zusammen mit dem oder den Reaktionsteil(en) und wenigstens einem Abtriebsteil in der Umesterungskolonne untergebracht. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone des Abtriebsteils wird ein Gemisch enthaltend Alkylenglykol, überschüssigem oder nicht umgesetztem Alkylencarbonat, Alkylalkohol, Dialkylcarbonat, Umesterungskatalysatoren und bei der Reaktion entstehende oder bereits in den Edukten enthaltene schwersiedende Verbindungen erhalten. Der Gehalt an leichtsiedenden Verbindungen, wie beispielsweise Dialkylcarbonat und Alkohol wird reduziert, wobei in Anwesenheit des Umesterungskatalysators unter Umständen weiteres Dialkylcarbonat und Alkylenglykol gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 50 bis 160°C. Es ist von Vorteil, die Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um abolute Druckangaben.

Bevorzugt wird das am Kopf der Umesterungskolonne in dem Verfahren zur Herstellung des Dialkylcarbonates entnommene Dampfgemisch enthaltend Dialkylcarbonat und Alkylalkohol nach Kondensation am Kopf der Umesterungskolonne ganz oder teilweise wenigstens einem weiteren Verfahrensschritt enthaltend wenigstens eine Destillationskolonne zur Trennung von Dialkylcarbonat und Alkylalkohol zugeführt.

Die Trennung des Dialkylcarbonats und des Alkylalkohols erfolgt bevorzugt destillativ in einer oder mehreren Destillationskolonnen oder in einer Kombination aus Destillation und Membrantrennung - im Folgenden als Hybridprozess - bezeichnet (siehe z. B. US-4,162,200 A, EP 581 115 B1, EP 592 883 B1 und WO 2007/096343A1).

Bilden Alkylalkohol und Dialkylcarbonat ein Azeotrop (z.B. Methanol und Dimethylcarbonat) so kann auch ein zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet werden. Besonders bevorzugt wird das Zweidruckverfahren oder ein Hybridverfahren angewendet.

Ganz besonders bevorzugt wird die Trennung des Dialkylcarbonats und des Alkylalkohols - selbst in dem Falle, dass das Dialkylcarbonat und der Alkylalkohol ein Azeotrop bilden - in einer einzelnen Destillationskolonne durchgeführt. Diese Destillationskolonne wird bei einem Druck betrieben, der höher ist als der Druck der Umesterungskolonne(n). Der Betriebsdruck der Destillationskolonne liegt im Bereich von 1 bis 50 bar, vorzugsweise zwischen 2 und 20 bar Am Sumpf der Destillationskolonne wird das nahezu reine Dialkylcarbonat entnommen und am Kopf ein Gemisch aus Dialkylcarbonat und Alkylalkohol. Dieses Gemisch wird der oder den Umesterungskolonne(n) ganz oder teilweise zugeführt. Wird das Verfahren zur Herstellung von Dialkylcarbonat mit einem Verfahren zur Herstellung von Diarylcarbonat, welches durch Umesterung dieses Dialkylcarbonates mit einer aromatischen Hydroxyverbindung gebildet wird, gekoppelt, so kann ein Teil des Gemisches aus Dialkylcarbonat und Alkylalkohol, welches am Kopf der Destillationskolonne entnommen wird, einem entsprechenden Aufarbeitungsschritt für Alkylalkohol und Dialkylcarbonat in der Verfahrensstufe zur Herstellung von Diarylcarbonat zugeführt werden.

In einer besonders bevorzugten Ausführung, wenn das Dialkylcarbonat und der Alkylalkohol ein Azeotrop bilden, ist dieser Aufarbeitungsschritt ein Zweidruckverfahren. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Alkylalkohol und Dialkylcarbonat ein Azeotrop, so weist das Destillat einer ersten Destillationskolonne des Verfahrensschritts zur Trennung von Dialkylcarbonat und Alkylalkohol vorzugsweise nahezu azeotrope Zusammensetzung auf. In diesem Fall wird dieses bevorzugt in einem Zweidruckverfahren wenigstens einer weiteren Destillationskolonne zugeführt die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Alkylalkohol. Man erhält als Sumpfprodukt dieser zweiten oder weiteren Destillationskolonne(n) Alkylalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Alkylalkohol und Dialkylcarbonat, wie beispielsweise Methanol und Dimethylcarbonat, verschiebt sich die azeotrope Zusammensetzung mit zunehmendem Druck zu höheren Alkylalkoholgehalten. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltene azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Alkylalkoholgehalten. Hierdurch ist es möglich, das in der Dialkylcarbonatkolonne erhaltene azeotrope Gemisch in ein Destillat mit nahezu azeotroper Zusammensetzung und nahezu reinen Alkylalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

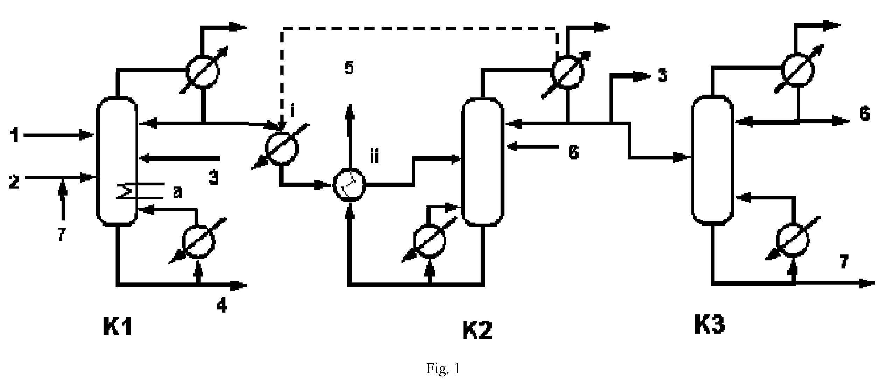

Eine beispielhafte Reaktionsführung bei der Trennung von Dialkylcarbonat und Alkylalkohol nach dem Zweidruckverfahren ist in Fig. 1 gezeigt.

Ein weiteres bevorzugtes Verfahren zur Trennung von Azeotropen aus Alkylalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Alkylalkohol und Dialkylcarbonat, wie beispielsweise Methanol und Dimethylcarbonat, erhält man bei Verwendung geeigneter Membranen mittels Pervarporation oder Dampfpermeation ein Alkylalkohol-reiches Gemisch als Permeat und ein an Alkylalkohol verarmtes Gemisch als Retentat. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Alkylalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltende dampfförmige Gemisch wird gegebenenfalls nach Überhitzung einer Dampfpermation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche Fraktion mit einem Alkylalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Alkylalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltende Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche dampfförmige Fraktion mit einem Alkylalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Alkylalkoholanteil erhält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervarporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Alkylalkohol erfolgt im Falle eines Hybridverfahrens besonders bevorzugt mittels einer Kombination aus Destillation und Dampfpermeation.

Die zur Trennung von Alkylalkohol und Dialkylcarbonat erforderliche Wärme wird bei einer Temperatur zwischen 100°C und 300°C, vorzugsweise zwischen 100°C und 230°C, und besonders bevorzugt zwischen 120°C und 200°C zugeführt.

Die Destillationskolonne(n) zur Aufreinigung des Dialkylcarbonats verfügen bevorzugt über einen Verstärkungsteil mit bevorzugt 5 bis 40 theoretischen Stufen zur Aufkonzentrierung des Alkylalkohols und einen Abtriebsteil mit bevorzugt 5 bis 40 theoretischen Stufen zur Aufkonzentrierung des Dialkylcarbonates.

Das Verfahren zur Herstellung von Dialkylcarbonat wird bevorzugt kontinuierlich durchgeführt.

In den Abbildungen bedeuten:
- K1: Umesterungskolonne
- K2: erste Destillationskolonne zur Trennung des Gemisches enthaltend Dialkylcarbonat und Alkylalkohol
- K3: zweite Destillationskolonne zur Trennung des Gemisches enthaltend Dialkylcarbonat und Alkylalkohol
- 1: Eduktstrom enthaltend Alkylencarbonat und/oder optional Katalysator
- 2: Eduktstrom enthaltend nahezu reinen Alkylalkohol
- 3: Eduktstrom enthaltend Alkylalkohol und Dialkylcarbonat
- 4: Strom enthaltend Alkylenglykol
- 5: Strom enthaltend aufgereinigtes Dialkylcarbonat
- 6: Strom enthaltend Dialkylcarbonat und Alkylalkohol
- 7: Strom enthaltend nahezu reinen Alkylalkohol
- 8: Strom enthaltend Extraktionsmittel (vorzugsweise Alkylencarbonat)
- 9: Strom enthaltend Extraktionsmittel (vorzugsweise Alkylencarbonat)
- 10: Strom enthaltend Extraktionsmittel (vorzugsweise Alkylencarbonat)
- i.: Wärmetauscher zur Vorwärmung des Feeds der Destillationskolonne zur Aufreinigung des Dialkylcarbonates, wobei die am Kopf dieser Destillationskolonne freiwerdende Kondensationswärme zur Vorwärmung verwendet wird
- ii: Wärmetauscher zur Vorwärmung des Feeds der Destillationskolonne zur Aufreinigung des Dialkylcarbonates, wobei dem Sumpfablauf, der nahezu reines Dialkylcarbonat enthält, Wärme entzogen wird
- a: Zwischenerhitzer

Fig. 1 beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Zweidruckdestillation in einer ersten (K2) und einer zweiten (K3) Destillationskolonne mit einem Zwischenerhitzer (a) in der Umesterungskolonne.

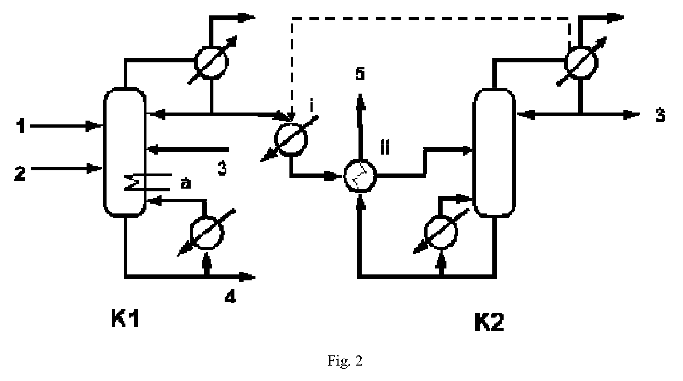

Fig. 2 beschreibt einen Umesterungsschritt von Alkylencarbonat und Alkylalkohol mittels Reaktivrektifikation in einer ersten Umesterungskolonne (K1), enthaltend einen Zwischenerhitzer (a), im Allgemeinen und die Aufarbeitung des am Kopf der Umesterungskolonne anfallenden Gemisches enthaltend Dialkylcarbonat und Alkylalkohol mittels einer einzelnen Destillationskolonne (K2).

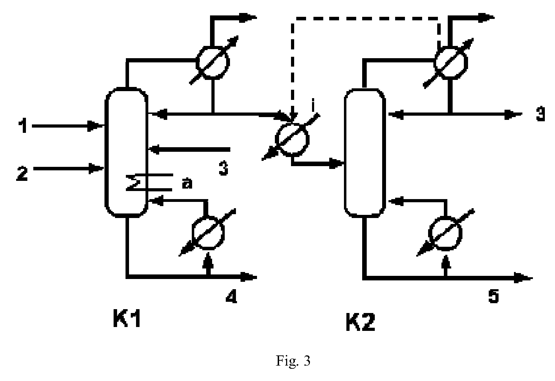

Fig. 3 wie Fig. 2, jedoch ohne Vorwärmung des Feeds der Destillationskolonne (K2) mittels deren Sumpfablauf

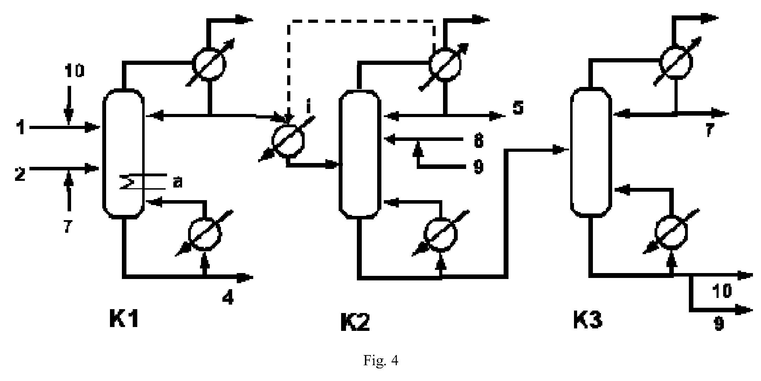

Fig. 4 beschreibt einen Umesterungsschritt von Alkylencarbonat und Alkylalkohol mittels Reaktivrektifikation in einer ersten Umesterungskolonne (K1), enthaltend einen Zwischenerhitzer (a), im Allgemeinen und die Aufarbeitung des am Kopf der Umesterungskolonne anfallenden Gemisches enthaltend Dialkylcarbonat und Alkylalkohol mittels Extraktivdestillation in einer ersten (K2) und einer zweiten (K3) Destillationskolonne, wobei das Alkylencarbonat vorzugsweise als Extraktionsmittel verwendet wird.

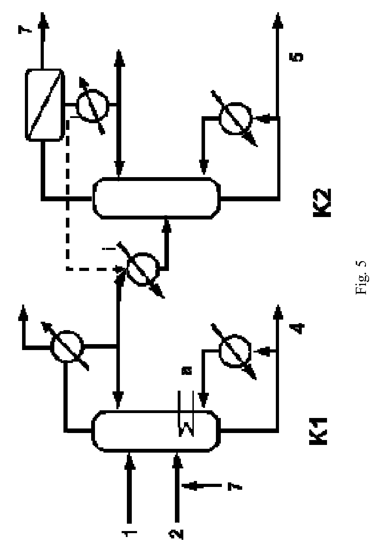

Fig. 5 beschreibt einen Umesterungsschritt von Alkylencarbonat und Alkylalkohol mittels Reaktivrektifikation in einer ersten Umesterungskolonne (K1), enthaltend einen Zwischenerhitzer (a), im Allgemeinen und die Aufarbeitung des am Kopf der Umesterungskolonne anfallenden Gemisches enthaltend Dialkylcarbonat und Alkylalkohol mittels Destillation und Dampfpermeation in einer Destillationskolonne (K2).

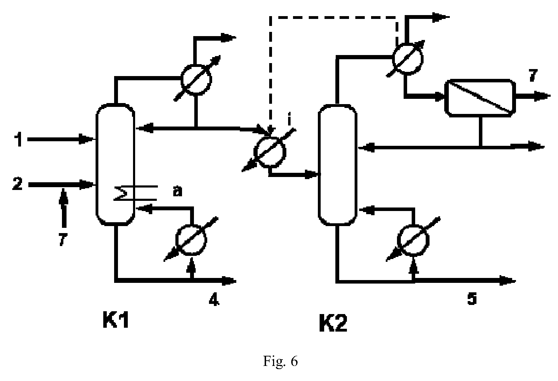

Fig. 6 beschreibt einen Umesterungsschritt von Alkylencarbonat und Alkylalkohol mittels Reaktivrektifikation in einer ersten Umesterungskolonne (K1), enthaltend einen Zwischenerhitzer (a), im Allgemeinen und die Aufarbeitung des am Kopf der Umesterungskolonne anfallenden Gemisches enthaltend Dialkylcarbonat und Alkylalkohol mittels Destillation und Pervaporation in einer Destillationskolonne (K2).

### Beispiele

Anhand eines Beispiels wird nun die bevorzugte Betriebsweise für das erfindungsgemäße Verfahren detailliert aufgezeigt. Beispiel 1 zeigt die bevorzugte Betriebsweise für die Umesterungskolonne. Dieses Beispiel soll auf keine Weise als Limitierung der Erfindung ausgelegt werden.

Der Vorteil dieser Erfindung, nämlich die Verringerung des Verbrauchs an frischem Heizdampf unter Einhaltung der hohen Produktqualität durch Installation einer technischen Vorrichtung zur Zwischenerhitzung, gegenüber anderen Betriebsarten ohne den bereits erwähnten Zwischenerhitzer oder bei einer ungünstigeren Platzierung des Zwischenverdampfers wird in den Vergleichsbeispielen dargestellt.

### Beispiel 1 (erfindungsgemäß)

Dieses Beispiel folgt den Bezeichnungen der Fig. 2. Eine reaktive Destillationskolonne (K1) bestehend aus einem Verstärkungsteil mit 9 theoretischen Stufen, einer Reaktionszone mit 25 Reaktionsböden (Holdup/Boden: 0,6 m³) und einem Abtriebsteil mit 4 theoretischen Stufen wird bei einem Druck am Kopf der Kolonne von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol (Strom 1) zudosiert. Zwischen dem 8. und 9. Reaktionsboden werden 21371 kg/h eines Dampfgemisches (Strom 3) mit 83,7 Gew.-% Methanol und 16,3 Gew.-% Dimethylcarbonat zugeführt. Zusätzlich dazu werden am unteren Ende der Reaktionszone 7123 kg/h eines Dampfgemisches (Strom 2) mit 99,5 Gew.-% Methanol, 0,41 Gew.-% Ethylenglykol und 672 ppm Dimethylcarbonat zugeführt.

Ein Partialkondensator kondensiert den Dampfstrom am Kopf der Kolonne (K1) bei 40 °C. Man erhält sowohl 6 kg/h dampfförmiges als auch 30644 kg/h flüssiges Destillat mit einer Zusammensetzung von 59 Gew.-% Methanol und 41 Gew.-% Dimethylcarbonat, das der Dimethylcarbonat-Aufreinigungskolonne zugeführt wird.

Im Abtriebsteil direkt oberhalb des Sumpfverdampfers der Umesterungskolonne (K1) ist eine Vorrichtung zur Zwischenerhitzung (Wärmetauscher a) mit einer Heizleistung von 2000 kW installiert, die mit Hilfe von Dampf auf einer Druckstufe von 1,5 bar als Heizmedium betrieben wird.

Man erhält 7019 kg/h flüssiges Sumpfprodukt (Strom 4), das hauptsächlich Ethylenglykol und unter anderem 477 ppm Ethylencarbonat beinhaltet. Der Sumpfverdampfer wird mit Heizdampf auf einer Druckstufe von 3 bar bei 102 °C betrieben.

Die nachfolgende Dialkylcarbonat-Aufreinigungskolonne (K2) besteht aus einem Verstärkungsteil mit 28 theoretischen Stufen und einem Abtriebsteil mit 11 theoretischen Stufen und wird bei einem Druck von 10 bar (absolut) am Kopf der Kolonne und einem Rücklaufverhältnis von 1,0 betrieben. Im unteren Kolonnenbereich wird zwischen der 27. und 28. theoretischen Trennstufe kontinuierlich die 30644 kg/h des Destillates der Umesterungskolonne enthaltend 59 Gew.-% Methanol und 41 Gew.-% Dimethylcarbonat zudosiert.

Ein Partialkondensator kondensiert den Dampfstrom am Kopf der Kolonne (K2) bei 137 °C. Man erhält sowohl 21 kg/h dampfförmiges als auch 21378 kg/h flüssiges Destillat (Strom 3) mit einer Zusammensetzung von 84 Gew.-% Methanol und 16 Gew.-% Dimethylcarbonat. Hierbei wird 10000 kW Wärmeleistung, die zur Dampferzeugung bei einer Druckstufe von 1,5 bar genutzt werden kann, abgeführt.

Man erhält 9245 kg/h flüssiges Sumpfprodukt (Strom 5) mit einer Zusammensetzung von 99,5 Gew.-% Dimethylcarbonat und 0,5 Gew.-% Methanol. Der Sumpfverdampfer wird mit Heizdampf auf einer Druckstufe von 16 bar bei 183 °C betrieben und hat eine Heizleistung von 10396 kW.
Ein Teil der am Kopf der Dialkylcarbonat-Aufreinigungskolonne gewonnenen Dampfmenge kann unter anderem zur Vorerhitzung des Feeds dieser Kolonne und für die Verdampfung des Zustroms (3) zur Umesterungskolonne (K1) genutzt werden. 2000 kW Dampfenergie werden für den Zwischenerhitzer der Umesterungskolonne verwendet.

Durch den Einsatz von 2000 kW aus der am Kopf der Dialkylcarbonat-Aufreinigungskolonne zurückgewonnenen Kondensationsenergie im Zwischenerhitzers der Umesterungskolonne ist für Betrieb des Sumpfverdampfers in der Umesterungskolonne nur noch eine Heizleistung von 939 kW, die über Heizdampf der Druckstufe 3 bar zur Verfügung gestellt wird, erforderlich.

### Beispiel 2 (Vergleich)

Es wird die gleiche reaktive Destillationskolonne (K1) und die gleiche Dialkylcarbonat-Aufreinigungskolonne (K2), wie im erfindungsgemäßen Beispiel 1 beschrieben, verwendet. Die Umesterungskolonne (K1) wird bei einem Druck am Kopf der Kolonne von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol zudosiert. Zwischen dem 8. und 9. Reaktionsboden werden 21371 kg/h eines Dampfgemisches mit 83,7 Gew.-% Methanol und 16,3 Gew.-% Dimethylcarbonat zugeführt. Zusätzlich dazu werden am unteren Ende der Reaktionszone 7123 kg/h eines Dampfgemisches mit 99,5 Gew.-% Methanol, 0,41 Gew.-% Ethylenglykol und 672 ppm Dimethylcarbonat zugeführt. Das Mengenverhältnis zwischen dem insgesamt zugeführten Methanol und dem Ethylencarbonat bleibt gegenüber Beispiel 1 konstant.

Ein Partialkondensator kondensiert den Dampfstrom am Kopf der Kolonne bei 40 °C. Man erhält sowohl 6 kg/h dampfförmiges als auch 30644 kg/h flüssiges Destillat mit einer Zusammensetzung von 59 Gew.-% Methanol und 41 Gew.-% Dimethylcarbonat, das weiteren Aufreinigungsschritten zugeführt wird.

Der Zwischenerhitzer (a) ist nicht in Betrieb.

Man erhält nun 7019 kg/h flüssiges Sumpfprodukt (Strom 4) aus der Umesterungskolonne (K1), das hauptsächlich Ethylenglykol und unter anderem 468 ppm Ethylencarbonat beinhaltet. Der Sumpfverdampfer der Umesterungskolonne wird mit Heizdampf auf einer Druckstufe von 3 bar bei 102 °C betrieben und erfordert nun eine Heizleistung von 2939 kW, mithin eine um 2000 kW höhere Heizleistung als im erfindungsgemäßen Beispiel.

### Beispiel 3 (Vergleich)

Es wird die gleiche reaktive Destillationskolonne (K1) und die gleiche Dialkylcarbonat-Aufreinigungskolonne (K2), wie im erfindungsgemäßen Beispiel 1 beschrieben, verwendet. Die Umesterungskolonne (K1) wird bei einem Druck am Kopf der Kolonne von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol zudosiert. Zwischen dem 8. und 9. Reaktionsboden werden 21371 kg/h eines Dampfgemisches mit 83,7 Gew.-% Methanol und 16,3 Gew.-% Dimethylcarbonat zugeführt. Zusätzlich dazu werden am unteren Ende der Reaktionszone 7123 kg/h eines Dampfgemisches mit 99,5 Gew.-% Methanol, 0,41 Gew.-% Ethylenglykol und 672 ppm Dimethylcarbonat zugeführt.

Ein Partialkondensator kondensiert den Dampfstrom am Kopf der Kolonne bei 40 °C. Man erhält sowohl 6 kg/h dampfförmiges als auch 30644 kg/h flüssiges Destillat mit einer Zusammensetzung von 59 Gew.-% Methanol und 41 Gew.-% Dimethylcarbonat, das weiteren Aufreinigungsschritten zugeführt wird.

Im 20. Reaktionsboden der Kolonne ist eine Vorrichtung zur Zwischenerhitzung mit einer Heizleistung von 2000 kW installiert, die mit Hilfe von Dampf auf einer Druckstufe von 1,5 bar als Heizmedium betrieben wird.

Man erhält nun 7019 kg/h flüssiges Sumpfprodukt, das hauptsächlich Ethylenglykol und unter anderem 1% Ethylencarbonat beinhaltet. Der Sumpfverdampfer wird mit Heizdampf auf einer Druckstufe von 3 bar bei 102 °C betrieben und erfordert eine Heizleistung von 939 kW. Die Verschiebung der Position des Zwischenerhitzers in der Umesterungskolonne führt zu einer drastischen Erhöhung des Gehaltes an Ethylencarbonat im flüssigen Sumpfprodukt (Strom 4) der Umesterungskolonne (K1).

## Patentansprüche

1. Verfahren zur Herstellung von Dialkylcarbonaten aus zyklischem Alkylencarbonat und Alkylalkohol in wenigstens einer Umesterungskolonne, enthaltend wenigstens eine Reaktionszone und wenigstens ein Verstärkungsteil, welches oberhalb der Reaktionszone angeordnet ist, ein Abtriebsteil, welches unterhalb der Reaktionszone angeordnet ist und wenigstens eine anschließende Destillationskolonne zur Aufreinigung des in der Umesterungskolonne gebildeten Dialkylcarbonates, enthaltend wenigstens ein Verstärkungsteil im oberen Teil der Kolonne und wenigstens ein Abtriebsteil im unteren Teil der Kolonne, **dadurch gekennzeichnet, dass**
eine technische Vorrichtung zur Erhitzung des Flüssigkeitsstromes in der Umesterungskolonne eingesetzt wird,
wobei die Temperatur Tz des Mediums zur Erhitzung des kolonneninternen Flüssigkeitsstromes kleiner als die Temperatur T_{SV} des Mediums ist, mit der der Sumpfverdampfer betrieben wird, und
die Energie des Mediums zur Erhitzung des kolonneninternen Flüssigkeitsstromes teilweise oder ganz in einem anderen chemischen Herstellverfahren zurückgewonnen wird,
wobei die technische Vorrichtung zur Erhitzung des Flüssigkeitsstromes oberhalb eines zu dieser wenigstens einen Umesterungskolonne gehörenden Sumpfverdampfers im Abtriebsteil angeordnet ist.

2. Verfahren nach Anspruch 1, **dadurch gekennzeichnet, dass** die Energie auf dem Temperaturniveau Tz durch Kondensation als Kondensationswärme ganz oder teilweise, direkt oder indirekt verfügbar gemacht wird.

3. Verfahren nach Anspruch 1 oder 2, **dadurch gekennzeichnet, dass** die technische Vorrichtung zur Erhitzung des kolonneninternen Flüssigkeitsstromes innerhalb oder außerhalb der Umesterungskolonne platziert ist.

4. Verfahren zur Herstellung von Dialkylcarbonat nach Anspruch 1 oder 3, **dadurch gekennzeichnet, dass** die Umesterung in wenigstens einer Umesterungskolonne in Gegenwart eines Katalysators im Gegenstrom so geführt wird, dass Alkylencarbonat in den oberen Teil der Kolonne und ein Dialkylcarbonat enthaltender Alkylalkohol, dessen Dialkylcarbonatgehalt bei 0,2 bis 30 Gew.% liegt, in den mittleren oder unteren Teil der Reaktionszone der wenigstens einen Umesterungskolonne eingeführt wird.

5. Verfahren nach Anspruch 4, **dadurch gekennzeichnet, dass** der wenigstens einen Umesterungskolonne ein weiterer Strom enthaltend nahezu reinen Alkylalkohol zugeführt wird, dessen Einführstelle unterhalb der Einführstelle des Dialkylcarbonat enthaltenden Alkylalkoholstromes angeordnet ist.

6. Verfahren nach Anspruch 5, **dadurch gekennzeichnet, dass** die Energie des Mediums auf dem Temperaturniveau Tz bei der Kondensation des Stoffgemisches am Kopf der Destillationskolonne(n) zur Aufreinigung des Dialkylcarbonates gewonnen wird.

## Claims

1. Process for preparing dialkyl carbonates from cyclic alkylene carbonate and alkyl alcohol in at least one transesterification column containing at least one reaction zone and at least one enrichment section arranged above the reaction zone, a stripping section arranged below the reaction zone and at least one subsequent distillation column for purifying the dialkyl carbonate formed in the transesterification column, containing at least one enrichment section in the upper part of the column and at least one stripping section in the lower part of the column, **characterized in that**
a technical device is used for heating the liquid stream in the transesterification column,
where the temperature T_{I} of the medium for heating the internal liquid stream in the column is less than the temperature T_{BV} of the medium by means of which the bottom vaporizer is operated, and
the energy of the medium for heating the internal liquid stream in the column is partly or entirely recovered in another chemical production process,
where the technical device for heating the liquid stream is arranged above a bottom vaporizer belonging to this at least one transesterification column in the stripping section.

2. Process according to Claim 1, **characterized in that** the energy at the temperature level T_{I} is made available, in its entirety or in part, directly or indirectly by condensation as heat of condensation.

3. Process according to Claim 1 or 2, **characterized in that** the technical device for heating the internal liquid stream in the column is positioned within or outside the transesterification column.

4. Process for preparing dialkyl carbonate according to Claim 1 or 3, **characterized in that** the transesterification is carried out in countercurrent in at least one transesterification column in the presence of a catalyst in such a way that alkylene carbonate is introduced into the upper part of the column and a dialkyl carbonate-containing alkyl alcohol having a dialkyl carbonate content of from 0.2 to 30% by weight is introduced into the middle or lower part of the reaction zone of the at least one transesterification column.

5. Process according to Claim 4, **characterized in that** a further stream containing virtually pure alkyl alcohol is fed into the at least one transesterification column at a point of introduction arranged below the point of introduction of the dialkyl carbonate-containing alkyl alcohol stream.

6. Process according to Claim 5, **characterized in that** the energy of the medium is recovered at the temperature level T_{I} in the condensation of the mixture at the top of the distillation column(s) for purifying the dialkyl carbonate.

## Revendications

1. Procédé de fabrication de carbonates de dialkyle à partir de carbonate d'alkylène cyclique et d'alcool alkylique dans au moins une colonne de transestérification, contenant au moins une zone de réaction et au moins une zone d'enrichissement, qui est agencée au-dessus de la zone de réaction, une zone de rectification, qui est agencée en dessous de la zone de réaction, et au moins une colonne de distillation ultérieure pour la purification du carbonate de dialkyle formé dans la colonne de transestérification, contenant au moins une zone d'enrichissement dans la partie supérieure de la colonne et au moins une zone de rectification dans la partie inférieure de la colonne, **caractérisé en ce que**
un dispositif technique pour le chauffage du courant de liquide dans la colonne de transestérification est utilisé,
la température T_{Z} du milieu pour le chauffage du courant de liquide dans la colonne étant inférieure à la température T_{SV} du milieu avec lequel l'évaporateur de fond est exploité, et
l'énergie du milieu pour le chauffage du courant de liquide dans la colonne étant récupérée en partie ou en totalité dans un autre procédé de fabrication chimique, le dispositif technique pour le chauffage du courant de liquide étant agencé dans la zone de rectification au-dessus d'un évaporateur de fond appartenant à ladite au moins une colonne de transestérification.

2. Procédé selon la revendication 1, **caractérisé en ce que** l'énergie est mise à disposition directement ou indirectement au niveau de température T_{Z} par condensation en totalité ou en partie en tant que chaleur de condensation.

3. Procédé selon la revendication 1 ou 2, **caractérisé en ce que** le dispositif technique pour le chauffage du courant de liquide dans la colonne est placé à l'intérieur ou à l'extérieur de la colonne de transestérification.

4. Procédé de fabrication de carbonate de dialkyle selon la revendication 1 ou 3, **caractérisé en ce que** la transestérification est réalisée à contre-courant dans au moins une colonne de transestérification en présence d'un catalyseur, de sorte que le carbonate d'alkylène soit introduit dans la partie supérieure de la colonne et un alcool alkylique contenant du carbonate de dialkyle, dont la teneur en carbonate de dialkyle est de 0,2 à 30 % en poids, dans la partie centrale ou inférieure de la zone de réaction de ladite au moins une colonne de transestérification.

5. Procédé selon la revendication 4, **caractérisé en ce qu'**un autre courant contenant de l'alcool alkylique pratiquement pur est introduit dans ladite au moins une colonne de transestérification, son emplacement d'alimentation étant agencé en dessous de l'emplacement d'alimentation du courant d'alcool alkylique contenant du carbonate de dialkyle.

6. Procédé selon la revendication 5, **caractérisé en ce que** l'énergie du milieu au niveau de température T_{Z} est obtenue lors de la condensation du mélange de matières à la tête de la ou des colonnes de distillation pour la purification du carbonate de dialkyle.
